(19) Europäisches Patentamt

European Patent Office

Office européen des brevets

(11) **EP 3 112 342 A1**

(12) **EUROPEAN PATENT APPLICATION**
published in accordance with Art. 153(4) EPC

(43) Date of publication:
04.01.2017 Bulletin 2017/01

(21) Application number: 15755313.2

(22) Date of filing: 26.02.2015

(51) Int Cl.:
*C07C 51/42* [(2006.01)]    *C07C 63/26* [(2006.01)]

(86) International application number:
PCT/JP2015/055717

(87) International publication number:
WO 2015/129831 (03.09.2015 Gazette 2015/35)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 27.02.2014 JP 2014037215

(71) Applicant: Mitsubishi Chemical Holdings
Corporation
Tokyo 100-8251 (JP)

(72) Inventor: IWAI, Hideo
Tokyo 100-8251 (JP)

(74) Representative: Merkle, Gebhard
Ter Meer Steinmeister & Partner
Patentanwälte mbB
Nymphenburger Straße 4
80335 München (DE)

(54) **METHOD FOR PRODUCING TEREPHTHALIC ACID**

(57) The present invention is aimed to provide a method for producing a terephthalic acid in which it is possible to subject the wastewater containing turbidity from a terephthalic acid production process to a membrane separation treatment continuously and stably over a long period of time and efficiently convert the separated water which is treated, into a high-concentration slurry to be reused for the terephthalic acid production process. The present invention relates to a method for producing terephthalic acid comprising a membrane separation step of subjecting a wastewater discharged from a terephthalic acid production process including an oxidation reaction step of oxidizing p-xylene in the presence of a metal compound to form crude terephthalic acid, to a membrane separation treatment in a treatment tank having a separation membrane provided therein, thereby separating it into a separated water and a membrane-permeated water, wherein aeration is performed from a lower part of the separation membrane.

EP 3 112 342 A1

**Description**

Technical Field

The present invention relates to a method for producing terephthalic acid. In detail, the present invention relates to a method for producing terephthalic acid including a wastewater treatment with good efficiency.

Background Art

As an industrial production method of terephthalic acid, a method in which an aromatic hydrocarbon having an alkyl group, such as p-xylene, that is a raw material, is subjected to an oxidation reaction in a liquid phase under pressure with a molecular oxygen-containing gas in a solvent containing an aliphatic carboxylic acid such as acetic acid, or the like in the presence of a catalyst of a metal compound composed mainly of cobalt, manganese, or the like, and/or a bromine compound, thereby producing terephthalic acid is widely adopted.

In terephthalic acid, in view of its commercial application, it is often required to increase the purity by reducing trace amounts of impurities as far as possible. In order to respond to such a requirement, as a purification method, there are adopted (A) a method in which in order to obtain terephthalic acid, a terephthalic acid slurry after the above-described oxidation reaction is separated and dried, and thereafter, the resultant is dissolved in a solvent such as water at a high temperature under a high pressure and hydrogenated in the presence of a catalyst, followed by separation; (B) a method in which in order to obtain terephthalic acid, a terephthalic acid slurry after the above-described oxidation reaction is additionally oxidized to advance an oxidation reaction of unreacted impurities, followed by separation and washing; and the like.

In the purification method, in order to completely dissolve sparingly water-soluble terephthalic acid in water, a large quantity of water is needed. In addition, it is needed to reduce the residual amount of the aliphatic carboxylic acid such as acetic acid, which is used as the solvent, or the like in the product as far as possible, a large quantity of water is also needed for washing.

As a result of such peculiar circumstances, a massive quantity of an industrial wastewater is discharged from a terephthalic acid production process. Nowadays, in particular, following the expansion of a production scale, terephthalic acid is produced in an amount of from 500,000 tons to 1,000,000 tons a year in a single production plant. Therefore, the amount of the wastewater which is drawn up from the underground or a neighboring water area and finally released to the public water area reaches as high as from 300 tons/hr to 500 tons/hr per single production plant. In addition, the production amount of terephthalic acid in the whole world is extremely massive and is likely expected to tend to expand in the future, and therefore, it may be considered that the wastewater from the terephthalic acid production process exerts an influence upon the environment to a non-negligible extent on a global scale. Thus, from the viewpoints of global environmental preservation, resource recycle, and the like, a requirement for appropriate means of treating the wastewater generated from the terephthalic acid production process is increasing.

As a technology of treating a wastewater containing a suspended solid (hereinafter sometimes referred to as "SS fraction"), which is generated in such a terephthalic acid production process, or a technology regarding reuse for the terephthalic acid production process after treating the wastewater, for example, Patent Literature 1 describes that after filtering a washing water generated during the terephthalic acid production, the resultant is allowed to pass through a cation exchange resin and an anion exchange resin in this order, thereby removing an unnecessary terephthalic acid solid, a metal catalyst, dissolved terephthalic acid, and a dissolved organic acid by-product.

In addition, Patent Literature 2 describes that an organic material-containing wastewater discharged from a production process of an aliphatic carboxylic acid or an ester thereof, or an aromatic carboxylic acid or an ester thereof, is subjected to a wet oxidation treatment using a solid catalyst and then separated into a non-permeated liquid and a permeated liquid by using a polyamide-based composite membrane (reverse osmosis membrane) having a high salt rejection.

Patent Literature 3, 4, or 5 describes that after reducing an organic material, a metal fraction, and the like in a wastewater discharged in a production process of terephthalic acid by combining plural wastewater treatment operations, the resultant is reused for the terephthalic acid production process.

Citation List

Patent Document

Patent Literature 1: JP-B-5-56329
Patent Literature 2: Japanese Patent No. 3372921
Patent Literature 3: Chinese Patent No. 100400432C
Patent Literature 4: Chinese Patent No. 100544429B
Patent Literature 5: Chinese Patent No. 101723531B

Summary of Invention

Problem that Invention is to Solve

However, according to the conventional methods up to date, it was difficult to subject the wastewater containing turbidity from a terephthalic acid production process to a membrane separation treatment continuously and stably over a long period of time and efficiently convert the separated water which is not permeated by the separation membrane, into a high-concentration slurry. For example, in the methods of the above-described patent literatures and the like, the treatment method of the filtered membrane-permeated water obtained by filtering the wastewater containing turbidity is described; however, how to treat the separated water is not described. Much more, the matter that the separated water is recovered as a high-concentration slurry and reused is not even suggested, and the production efficiency of terephthalic acid was very bad. In addition, in view of the fact that the turbidity of the wastewater in the neighborhood of the separation membrane is not lowered, it was difficult to perform the membrane separation treatment stably over a long period of time, the life of the separation membrane was short, and the frequency of interchange of the separation membrane was high.

Under such circumstances, an object of the present invention is to provide a method for producing terephthalic acid with enhanced production efficiency, in which a slurry is efficiently recovered from an SS fraction-containing wastewater discharged from a terephthalic acid production process, and furthermore, the slurry is reused for the terephthalic acid production process.

Furthermore, another object of the present invention is to provide a method for producing terephthalic acid, in which a slurry is efficiently recovered from an SS fraction-containing wastewater discharged from a terephthalic acid production process to reduce an SS concentration of the wastewater in the neighborhood of a separation membrane for performing filtration of the wastewater, thereby making it possible to undergo a wastewater treatment continuously and stably over a long period of time.

Means for Solving Problem

The present inventors analyzed the conventional technologies and made extensive and intensive investigations. As a result, it has been found that the following invention is in agreement with the foregoing objects, leading to accomplishment of the present invention.

Specifically, the gist of the present invention resides in the following [1] to [13].

[1] A method for producing terephthalic acid comprising a membrane separation step of subjecting a wastewater discharged from a terephthalic acid production process including an oxidation reaction step of oxidizing p-xylene in the presence of a catalyst to form crude terephthalic acid, to a membrane separation treatment in a treatment tank having a separation membrane provided therein, thereby separating it into a membrane-permeated water and a separated water which is not permeated by the separation membrane, wherein

aeration is performed from a lower part of the separation membrane.

[2] The method for producing terephthalic acid described in the above [1], wherein the membrane separation step includes a membrane separation treatment step and a step of precipitating a slurry.

[3] The method for producing terephthalic acid described in the above [1] or [2], wherein a temperature of the wastewater to be subjected to a membrane separation treatment is 30°C or higher and 60°C or lower.

[4] The method for producing terephthalic acid described in any one of the above [1] to [3], wherein a linear velocity of a flowing of the wastewater on the surface of the separation membrane is 50 m/hr or more and 200 m/hr or less.

[5] The method for producing terephthalic acid described in any one of the above [1] to [4], wherein a suspended solid (SS) concentration of the wastewater to be subjected to a membrane separation treatment is 1,000 mg/L or more and 15,000 mg/L or less.

[6] The method for producing terephthalic acid described in any one of the above [1] to [5], wherein the slurry is recovered from the separated water.

[7] The method for producing terephthalic acid described in the above [6], wherein the recovered slurry is used as a process water of the terephthalic acid production process.

[8] The method for producing terephthalic acid described in any one of the above [1] to [7], wherein the aeration is performed by an air diffuser.

[9] The method for producing terephthalic acid described in the above [8], wherein an air discharge port is provided in the air diffuser, and an aperture of the air discharge port is $\phi$1 mm or more and $\phi$10 mm or less.

[10] The method for producing terephthalic acid described in any one of the above [1] to [9], wherein the separation membrane is a membrane filter.

[11] The method for producing terephthalic acid described in any one of the above [1] to [10], wherein a pore diameter of the separation membrane is from 0.01 $\mu$m to 10 $\mu$m.

[12] The method for producing terephthalic acid described in any one of the above [1] to [11], wherein the separation membrane is a hollow fiber membrane.

[13] The method for producing terephthalic acid described in any one of the above [1] to [12], wherein a membrane separation treatment amount (m/day) per unit membrane area ($m^2$) in the separation membrane is 0.5 or more and 5 or less.

Advantageous Effects of Invention

According to the present invention, by efficiently recovering a high-concentration slurry from an SS fraction-containing wastewater discharged from a terephthalic acid production process, it has become possible to reduce the SS concentration of the wastewater in the neighborhood of a separation membrane for performing filtration of the wastewater, thereby making it possible to undergo a wastewater treatment continuously and stably over a long period of time. Furthermore, by reusing the high-concentration slurry for the terephthalic acid production process, it has become possible to produce terephthalic acid with enhanced production efficiency.

Brief Description of Drawings

Fig. 1 is a flowchart showing an example of a first representative production process of terephthalic acid.
Fig. 2 is a flowchart showing an example of a second representative production process of terephthalic acid.
Fig. 3 is a flowchart showing an example of a first representative membrane separation step.
Fig. 4 is a flowchart showing an example of a second representative membrane separation step.

Description of Embodiments

While the embodiments of the present invention are hereunder described in detail, the description of constituent features as described below is an example (representative example) of the embodiments of the present invention, and the present invention is not limited to the following contents, so long as it does not exceed the gist thereof. In addition, it should be construed that a description regarding "from A to B" expressing a numerical value range means "A or more and B or less" unless otherwise indicated.

<A. Production method of terephthalic acid>

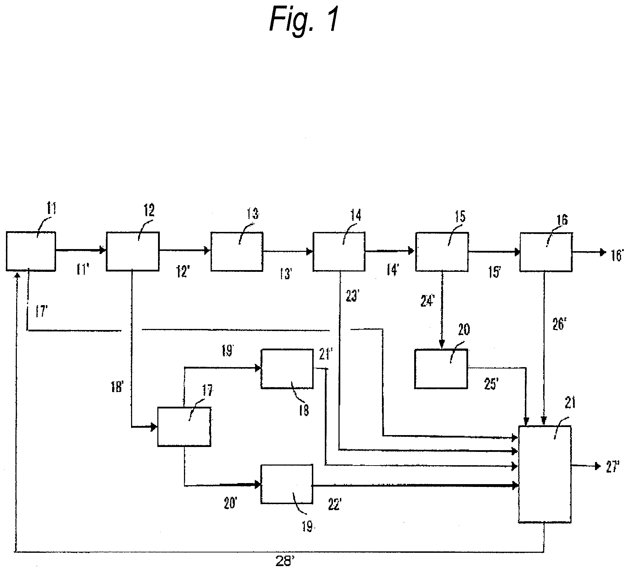

Fig. 1 is a diagram for explaining a step flow and a material flow in a first representative production method of terephthalic acid. The first representative production process of terephthalic acid includes the following 8 steps.
(1) Step of oxidizing p-xylene in aqueous acetic acid to obtain a crude terephthalic acid slurry (hereinafter sometimes referred to as "oxidation reaction step (1)");
(2) Step of subjecting the crude terephthalic acid slurry to solid-liquid separation to recover a crude terephthalic acid cake (hereinafter sometimes referred to as "first solid-liquid separation step (2)");
(3) Step of dissolving the crude terephthalic acid cake in water, followed by performing a hydrogenation treatment (hereafter sometimes referred to as "hydrogenation treatment step (3)");
(4) Step of crystallizing the hydrogenation treatment liquid to obtain a terephthalic acid slurry (hereinafter sometimes referred to as "crystallization step (4)");
(5) Step of subjecting the terephthalic acid slurry to solid-liquid separation to obtain a terephthalic acid cake and a separated mother liquor (hereinafter sometimes referred to as "second solid-liquid separation step (5)");
(6) Step of drying the terephthalic acid cake to obtain terephthalic acid (hereinafter sometimes referred to as "drying step (6)");
(7) Step of subjecting the whole or a part of the wastewaters discharged from the above-described steps (1) to (6) to solid-liquid separation with a separation membrane to obtain a membrane-permeated water (hereinafter sometimes referred to as "membrane separation step (7)"); and

(8) Step of separating the whole or a part of the membrane-permeated water obtained from the step (7) into a treated water and a mother liquor by a reverse osmosis membrane (hereinafter sometimes referred to as "reverse osmosis membrane treatment step (8)").
In this specification, the "terephthalic acid production process" means the steps relative to the production of terephthalic acid excluding the steps of wastewater treatment and the like, and for example, it is corresponding to the steps (1) to (6) in the above-described <A. Production method of terephthalic acid>.

(Oxidation reaction step (1))

The oxidation reaction step (1) is an oxidation reaction step of oxidizing p-xylene in aqueous acetic acid to obtain a crude terephthalic acid slurry 11'. First of all, p-xylene and a solvent containing acetic acid and the like are mixed and sent to an oxidation reaction apparatus 11, and the p-xylene is oxidized with molecular oxygen in a solvent in the presence of a catalyst. Examples of the catalyst include a metal compound of a transition metal such as cobalt and manganese and/or a hydrogen halide such as hydrobromic acid, a bromine compound, and the like. Specifically, examples of the metal compound include cobalt acetate, manganese acetate, and the like. According to this, the crude terephthalic acid slurry 11' is produced and sent to the first solid-liquid separation step (2).
A reaction temperature is generally from 180°C to 230°C, and preferably from 190°C to 210°C. As for a reaction pressure, a pressure under which the mixture may keep a liquid phase thereof at least at the reaction temperature, or more is needed, and specifically, it is preferably from 0.3 MPa to 10 MPa (absolute pressure), and more preferably from 1 MPa to 3 MPa (absolute pressure).

(First solid-liquid separation step (2))

The first solid-liquid separation step (2) is a step of subjecting the crude terephthalic acid slurry 11' to solid-liquid separation in a first solid-liquid separation apparatus 12 to recover a crude terephthalic acid cake, which is then washed and dried to obtain crude terephthalic acid 12'. Examples of a method of performing the solid-liquid separation may include a method of applying the crude terephthalic acid slurry 11' as it is to a solid-liquid separator, and the like. Incidentally, the crude terephthalic acid slurry 11' is in a pressurized state, and therefore, if the pressure is reduced, the dissolved crude terephthalic acid is deposited. For this reason, the slurry 11' may be sent to a crystallization tank (not illustrated) to perform bursting and cooling and after depositing the dissolved crude terephthalic acid, applied to a solid-liquid separator. Incidentally, it is meant by the terms "bursting and cooling" that a target liquid is introduced into the crystallization tank set up under a pressure condition lower than the pressure of the target liquid, and by performing bursting in the crystallization tank, the resultant is cooled by means of expansion and vaporization of the solvent component.

The thus obtained crude terephthalic acid cake is washed with acetic acid, water, or a mixture of acetic acid and water, or the like and then dried, thereby obtaining the crude terephthalic acid 12'. The obtained crude terephthalic acid 12' contains 4-carboxybenzaldehyde (hereinafter sometimes as "4CBA") that is an oxidation intermediate, and the like as impurities. In order to remove such impurities, the crude terephthalic acid 12' is sent to the subsequent hydrogenation treatment step (3).

(Hydrogenation treatment step (3))

The hydrogenation treatment step (3) is a step of dissolving the terephthalic acid 12' in water and performing a reduction treatment by means of hydrogenation in a hydrogenation treatment apparatus 13. By going through the hydrogenation treatment step (3), the 4CBA that is an impurity is reduced to form p-toluic acid. Since the p-toluic acid is higher in water solubility than terephthalic acid, it can be separated in the second solid-liquid separation step (5) as described later. Incidentally, the separated p-toluic acid is returned to the oxidation reaction step (1) and used as a raw material of terephthalic acid. A hydrogenation treatment liquid 13' produced in the hydrogenation treatment step (3) is sent to the subsequent crystallization step (4).

(Crystallization step (4))

The crystallization step (4) is a step of crystallizing the hydrogenation treatment liquid 13' to obtain a terephthalic acid slurry 14' in a crystallization apparatus 14. Examples of a crystallization method may include a method by evaporation removal of water that is the solvent and cooling, a method of performing bursting and cooling, and the like. In this step, as described above, the p-toluic acid is high in water solubility, and therefore, the majority thereof remains in a dissolved state in the solvent without being deposited. Thus, p-toluic acid and terephthalic acid can be separated from each other in the subsequent second solid-liquid separation step (5).

(Second solid-liquid separation step (5))

The second solid-liquid separation step (5) is a step of subjecting the terephthalic acid slurry 14' to solid-liquid separation to separate it into a terephthalic acid cake 15' and a terephthalic acid-separated mother liquor 24' in a second solid-liquid separation apparatus 15. As for the separation, a known method such as filtration and centrifugation can be adopted. The mother liquor 24' attaches to the separated terephthalic acid cake, and the impurities dissolved in the mother liquor, and the like lower the quality, and therefore, the terephthalic acid cake 15' is washed. The terephthalic acid cake 15' obtained by washing is sent to the subsequent drying step (6).

Here, the terephthalic acid-separated mother liquor 24' contains the impurities such as p-toluic acid, the catalyst, terephthalic acid, and the like, and therefore, the p-toluic acid and terephthalic acid, and the like are deposited and recovered by cooling in a recovery apparatus 20 of p-toluic acid, etc. The recovered p-toluic acid and terephthalic acid, and the like are returned to the oxidation reaction step (1). Meanwhile, after a heavy metal catalyst of cobalt, manganese, etc., and the like are recovered in a recovery apparatus (not illustrated), a separated mother liquor 25' of p-toluic acid, etc. is sent to the membrane separation step (7).

(Drying step (6))

The drying step (6) is a step of drying the terephthalic acid cake 15' to obtain terephthalic acid 16' in a drying apparatus 16. In drying, a pressurized dryer by bursting and evaporation, a usual fluidized dryer, and the like can be used.

(Wastewater from the oxidation reaction step (1))

Following the molecular oxygen-containing gas to be introduced into the oxidation reaction apparatus 11 for the oxidation reaction, a high-temperature high-pressure gas containing unreacted p-xylene, etc. as well as acetic acid as the solvent, water produced as a by-product, methyl acetate, methyl bromide, a reaction intermediate, carbon monoxide, and the like are discharged from the oxidation reaction step (1). By-product energy is recovered as electrical and heat energy from the high-temperature high-pressure gas, and furthermore, acetic acid, methyl acetate, and the like are recovered, and methyl bromide and the like are then subjected to a combustion treatment. After an exhaust gas after the combustion treatment is subjected to contact washing with alkali water or the like, the gas is released to the air, and an oxidization exhaust gas washing water 17' is sent to a treatment tank 21 having a separation membrane provided therein (hereinafter sometimes referred to as "treatment tank").

(Wastewater from the first solid-liquid separation step (2))

A mother liquor having been subjected to solid-liquid separation is generated from the first solid-liquid separation step (2). 50% by weight or more of the mother liquor is returned to the oxidation reaction step (1) and reused. Although a ratio of reusing the mother liquor is preferably 70% by weight or more, it is needed to purge a part of the mother liquor for the purpose of preventing a lowering of the quality to be caused due to accumulation of impurities within the system. For that reason, the ratio of reusing the mother liquor is 95% by weight or less, and preferably 90% by weight or less. An oxidation reaction mother liquor (purged fraction) 18' which is not submitted to the reuse is evaporated and concentrated in a solvent recovery apparatus 17 and separated into an evaporated solvent 19' and a mother liquor concentrated slurry 20' containing an active component.

The mother liquor concentrated slurry 20' is sent to a catalyst recovery/regeneration apparatus 19, whereby the catalyst is recovered/regenerated. After subjecting the recovered/regenerated catalyst to solid-liquid separation, a regenerated catalyst recovered and separated mother liquor 22' is sent together with a washing wastewater generated from a deodorization tower to an aerobic activated sludge processing apparatus (not illustrated). Meanwhile, the evaporated solvent 19' is sent to a recovery apparatus 18 of acetic acid, etc., the acetic acid is concentrated and recovered in a dehydration tower (not illustrated), and subsequently, methyl acetate is recovered in a distillation tower (methyl acetate recovery tower) (not illustrated). A part of a remaining bottom liquid 21' in the methyl acetate recovery tower is sent to the treatment tank 21.

Incidentally, vent gases generated in other steps than the oxidation reaction step (1) and first solid-liquid separation step (2) are collectively washed in a washing tower (not illustrated) and sent together with the oxidation exhaust gas washing water 17' to the treatment tank 21.

(Wastewater from the hydrogenation treatment step (3))

Although p-toluic acid and the like are generated from the hydrogenation treatment step (3), as described above, the p-toluic acid and the like can be separated in the crystallization step (4) and sent to the treatment tank 21 from the crystallization step (4).

(Wastewater from the crystallization step (4))

A condensed water 23' generated at the time of crystallization, which is obtained by cooling a vapor generated during crystallization by means of bursting and cooling or reduced pressure cooling, is generated from the crystallization step (4). The condensed water 23' generated at the time of crystallization contains organic materials such as p-toluic acid and is sent to the treatment tank 21.

(Wastewaters from the second solid-liquid separation step (5) and drying step (6))

In the second solid-liquid separation step (5), not only the terephthalic acid cake 15' is subjected to solid-liquid separation, but also the terephthalic acid-separated mother liquor 24' is generated. The mother liquor 24' is sent to the recovery apparatus 20 of p-toluic acid, etc., and the p-toluic acid and terephthalic acid, and the like dissolved in the mother liquor 24' are deposited by means of bursting and cooling and subjected to filtration and separation. The cake deposited and separated from the terephthalic acid-separated mother liquor 24' is converted into a slurry, which is then returned to the oxidation reaction step (1). The transition metal contained in the remaining mother liquor after deposition and separation, such as cobalt and manganese, is recovered in a metal recovery apparatus (not illustrated), and the remaining separated mother liquor 25' of p-toluic acid, etc. is then cooled and sent to the treatment tank 21.

Besides, a vent gas washing water 26' generated during collectively treating the vent gases with a scrubber is sent from the second solid-liquid separation step (5) and drying step (6) to the treatment tank 21.

The present inventors made investigations regarding the production method of terephthalic acid including the treatment method of the wastewaters discharged from the steps (1) to (6). As a result, in a membrane separation step of subjecting the wastewater in a treatment tank having a separation membrane provided therein to a membrane separation treatment, thereby separating it into a separated water and a membrane-permeated water, by regulating a temperature of the wastewater to be subjected to a membrane separation treatment within a specified range and performing the membrane separation treatment while allowing the wastewater to flow on the surface of the separation membrane, to efficiently recover a high-concentration slurry from the wastewater, it has become possible to reduce an SS concentration of the wastewater in the neighborhood of the separation membrane for performing filtration of the wastewater, thereby making it possible to undergo a wastewater treatment continuously and stably over a long period of time. Furthermore, it has been found that by reusing the recovered high-concentration slurry for the terephthalic acid production process, terephthalic acid with enhanced production efficiency can be produced.

(Membrane separation step (7))

The membrane separation step (7) includes a membrane separation treatment step in which in the treatment tank 21, a part or the whole of the wastewater discharged from each of the above-described steps is subjected to a membrane separation treatment by a separation membrane, thereby separating it into a separated water and a membrane-permeated water 27'. The membrane separation step (7) may include a step of precipitating the slurry in the wastewater. Since the wastewater is a wastewater from the terephthalic acid production process, it contains organic materials and inorganic materials.

As the organic materials contained in the wastewater, terephthalic acid is contained generally in an amount of from 1,000 ppm by weight to 5,000 ppm by weight, p-toluic acid is contained generally in an amount of from 500 ppm by weight to 5,000 ppm by weight, and benzoic acid is contained generally in an amount of from 50 ppm by weight to 500 ppm by weight. Furthermore, as the inorganic materials, for example, a compound of a metal used as the catalyst, such as cobalt and manganese, is contained, and cobalt is contained generally in an amount of from 1 ppm by weight to 50 ppm by weight in terms of a metal component, and manganese is contained generally in an amount of from 1 ppm by weight to 50 ppm by weight in terms of a metal component.

A temperature of the wastewater to be subjected to a membrane separation treatment in the production method of terephthalic acid of the present invention is preferably 30°C or higher and 60°C or lower. An upper limit of the temperature is more preferably 57°C, and still more preferably 55°C, and a lower limit thereof is more preferably 35°C, and still more preferably 40°C.

Since the wastewater discharged from each of the above-described steps is generally high in temperature, it is preferable to adjust it at the temperature of a wastewater and then perform the membrane separation. When the temperature of the wastewater to be subjected to a membrane separation treatment falls within the foregoing range, it becomes possible to increase a precipitation speed of the slurry toward a lower part within the treatment tank, and it becomes possible to reduce the SS concentration in the wastewater, effectively recover the slurry, and effectively reuse the recovered slurry. As a method of adjusting the temperature of the wastewater to be subjected to a membrane separation treatment to the foregoing range, for example, a method in which a cooling apparatus is installed in a liquid sending piping of the wastewater to the treatment tank, or a temperature adjuster is installed in the treatment tank, thereby controlling the internal temperature, may be considered.

Furthermore, the wastewater is subjected to a membrane separation treatment while allowing it to flow on the surface of the separation membrane. A solid attached to the separation membrane can be dispersed in the wastewater by means of the flowing.

Incidentally, the flowing of the wastewater on the surface of the separation membrane means that the wastewater flows in parallel to the membrane surface. The flowing direction is preferably an upper direction relative to the membrane surface. Although a technique for the flowing is not limited, examples thereof include flowing by aeration from a lower part of the separation membrane, flowing due to convection by a water feed pump, flowing by an agitation blade, etc., and the like. Of those, according to aeration from a lower part of the separation membrane, dispersion of a solid attached to the separation membrane into the wastewater can be efficiently performed. Furthermore, the solid is entrained by a downward flow generated by in-tank convection due to the aeration and precipitated as a slurry in a lower part within the treatment tank, and it becomes possible to effectively recover the slurry and effectively reuse the recovered slurry. Furthermore, a solid concentration in the slurry precipitated by the downward flow increases, the turbidity of the wastewater to be subjected to a membrane separation treatment is reduced, and it becomes possible to perform the membrane separation treatment continuously over a long period of time.

Incidentally, as for the recovery of the slurry, it is preferable to extract the slurry precipitated in the treatment tank from the bottom of the treatment tank because redispersion of the precipitated slurry into the separated water is suppressed.

It is preferable to perform the aeration by an air diffuser because the solid concentration in the slurry can be made high. Furthermore, the air diffuser is provided with an air discharge port. An aperture of the air discharge port is preferably φ1 mm or more and φ10 mm or less, and a lower limit thereof is more preferably φ2 mm, and still more preferably φ3 mm. An upper limit thereof is more preferably φ9 mm, and still more preferably φ8 mm. By controlling the aperture of the air discharge port to the upper limit or less, there is a possibility that redispersion of the precipitated slurry into the separated water is suppressed, whereas by controlling it to the lower limit or more, it becomes possible to increase the concentration of the solid in the slurry. Furthermore, it is preferable that the air diffuser is provided with an air diffusion pipe. Although the air diffusion pipe may be a single pipe, it is preferable to install plural pipes. Incidentally, though there is a method in which in addition to the aeration, an agitation blade or the like is installed within the treatment tank, and the wastewater is subjected to a membrane separation treatment while allowing the wastewater to flow by agitation, there is a possibility that the precipitation of the slurry is disturbed, and therefore, only the aeration is preferable.

A linear velocity of the flowing of the wastewater on the surface of the separation membrane is preferably from 50 m/hr to 200 m/hr, and more preferably from 60 m/hr to 150 m/hr. By controlling the linear velocity to the upper limit or less, there is a possibility that redispersion of the precipitated slurry is suppressed, whereas by controlling it to the lower limit or more, it becomes possible to remove a solid attached to the separation membrane and disperse the solid in the wastewater.

An SS concentration of the wastewater to be subjected to a membrane separation treatment is preferably 1,000 mg/L or more and 15,000 mg/L or less. A lower limit of the SS concentration is more preferably 2,000 mg/L, and still more preferably 3,000 mg/L, and an upper limit thereof is more preferably 13,000 mg/L, and still more preferably 11,000 mg/L. By allowing the SS concentration to fall within the foregoing appropriate range, the solid concentration in the slurry precipitated in a lower part of the treatment tank becomes high, and it becomes possible to recover the slurry and effectively reuse the recovered slurry. Furthermore, the turbidity of the wastewater to be subjected to a membrane separation treatment is appropriately reduced, and it becomes possible to perform the membrane separation treatment continuously over a long period of time.

Incidentally, the SS concentration of the wastewater means a concentration of the material suspended in the wastewater and is analyzed by a method described in JIS K0102.

By recovering the slurry within the treatment tank, it becomes possible to reduce the turbidity of the wastewater to be treated with a separation membrane. Thus, it becomes possible to perform the membrane separation treatment continuously over a long period of time without applying a load against the separation membrane. Furthermore, the solid contained in the slurry is a component obtained from the production process of terephthalic acid, and specifically, it includes p-toluic acid, terephthalic acid, benzoic acid, and the like. In the light of the above, it is preferable to use the recovered slurry as a process water for the terephthalic acid production process, and specifically, it is preferable to reuse the process water in the oxidation reaction step. By reusing the process water, there is a possibility that the production efficiency of terephthalic acid may be enhanced, and the production costs may be reduced.

Examples of the kind of the separation membrane which is used for the production method of the present invention include cellulose, a glass fiber filter, a membrane filter, and the like. Above all, a membrane filter is preferable because it is a membrane in which a pore diameter is uniform, and the membrane separation treatment may be performed efficiently and stably over a long period of time.

The pore diameter of the separation membrane is preferably from 0.01 $\mu$m to 10 $\mu$m, more preferably from 0.01 $\mu$m to 5 $\mu$m, and still more preferably from 0.05 $\mu$m to 2 $\mu$m. When the pore diameter is too small, there is a possibility that the membrane separation treatment ability is lowered. In addition, if a differential pressure is increased for the purpose of enhancing the treatment amount, there may be the case where a life of the separation membrane is shortened. When the pore diameter is too large, the recovery amount of the slurry is not obtained, the amount of the slurry to be submitted to the reuse is small, and there is a possibility that an enhancement of the production efficiency of terephthalic acid is not obtained.

Examples of a method of installing the separation membrane within the treatment tank include a suspension mode from an upper part of the treatment tank, a horizontal mounting mode of installing the separation membrane in a planar state in the inside of the treatment tank, and the like. Of these, a suspension mode is more preferable because the efficiency of the membrane separation treatment becomes favorable. As for a shape of the separation membrane, there are exemplified a flat membrane, a hollow fiber membrane, a tubular membrane, and a spiral membrane. However, a hollow fiber membrane is preferable because it has a large membrane area per unit volume, and therefore, it becomes possible to perform the membrane separation treatment efficiently.

A membrane separation treatment amount (m/day) per unit membrane area ($m^2$) in the separation membrane is preferably 0.5 or more and 5 or less, and more preferably 2 or more and 5 or less. When the membrane separation treatment amount of the separation membrane is too small, the area where the separation membrane is installed increases, and there is a possibility that the installation costs become large. By allowing the membrane separation treatment amount to fall within an appropriate range, it becomes possible to perform the membrane separation treatment continuously and efficiently.

As for the material of the separation membrane, for example, various materials capable of being formed into a shape of the separation membrane, such as polyolefin-based, polyvinyl alcohol-based, polysulfone-based, polyacrylonitrile-based, and fluorine-based resins, can be used. Examples thereof include polyethylene, polypropylene, polyvinylidene fluoride, polytetrafluoroethylene, polysulfone, and the like.

In particular, as for surface characteristics of the separation membrane, it is suitable to use a resin having strong hydrophobicity, and fluorine-based resins are especially preferable. Among the fluorine-based resins, it is more preferable to use a vinylidene fluoride resin from the standpoints of shape-forming properties into a membrane, chemical resistance, and the like. Here, examples of the vinylidene fluoride resin include, in addition to a homopolymer of vinylidene fluoride, a copolymer of vinylidene fluoride and a monomer which is copolymerizable with vinylidene fluoride. Examples of the copolymerizable monomer include vinyl fluoride, tetrafluoroethylene, trifluoroethylene, hexafluoropropylene, and the like.

Incidentally, the separation membrane may be used as a membrane module 22 as an aggregate, and specific examples thereof include "STERAPORE SADF" (a registered trademark) (manufactured by Mitsubishi Rayon Co., Ltd.), "MEMBRAY" (a registered trademark) (manufactured by Toray Industries, Inc.), "MICROZA" (a registered trademark) (manufactured by Asahi Kasei Chemicals Corporation), and the like. As for the membrane separation treatment method, dead-end filtration (also called "normal flow filtration") which is performed while accumulating the solid on the usual membrane surface and cross-flow filtration in which by creating a flow parallel to the membrane surface and making it orthogonal to the membrane permeation direction, a solid is always redispersed are generally performed, and the filtration may be selected depending upon the use purpose.

In the case of an ultrafiltration membrane (UF membrane), a microfiltration membrane (MF membrane), a reverse osmosis membrane (RO membrane), or the like, a cross-flow mode is frequently performed. However, a parallel flow to the membrane and a flow toward the inside of the membrane are orthogonal to each other, and if it is intended to increase the treatment efficiency, a state in which the accumulation amount of the solid on the membrane surface increases, whereby the treatment speed and the treatment efficiency are lowered cannot be avoided. At this time, in many cases, a pretreatment operation of separating and removing particles suspended in a slurry state in advance, or other operation is needed.

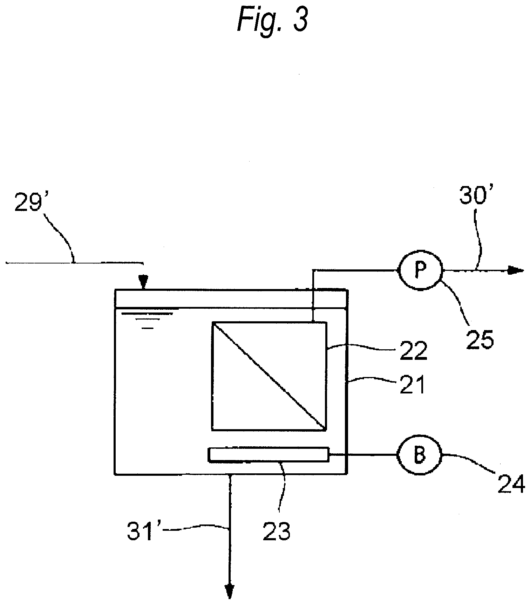

Next, the operation is specifically described based on flows. Fig. 3 is a diagram showing a first representative membrane separation step flow of a wastewater and a material flow in the production method of terephthalic acid. The first representative membrane separation step has the following constitutions.

(i) A wastewater 29' discharged from each of the steps in the production method of terephthalic acid is sent to the treatment tank 21.
(ii) The sent wastewater is separated into a separated water and a membrane-permeated water 30' by the membrane module 22.
(iii) The membrane-permeated water 30' is sent to the reverse osmosis membrane treatment step by a pump 25.
(iv) Aeration is performed from a lower part of the membrane module through an air diffusion pipe 23 by a blower 24.
(v) A slurry 31' precipitated in the treatment tank 21 is recovered.
(vi) The recovered slurry is sent to the oxidation reaction step.

A temperature of the wastewater to be subjected to a membrane separation treatment in the above-described steps is 40°C or higher and 60°C or lower.

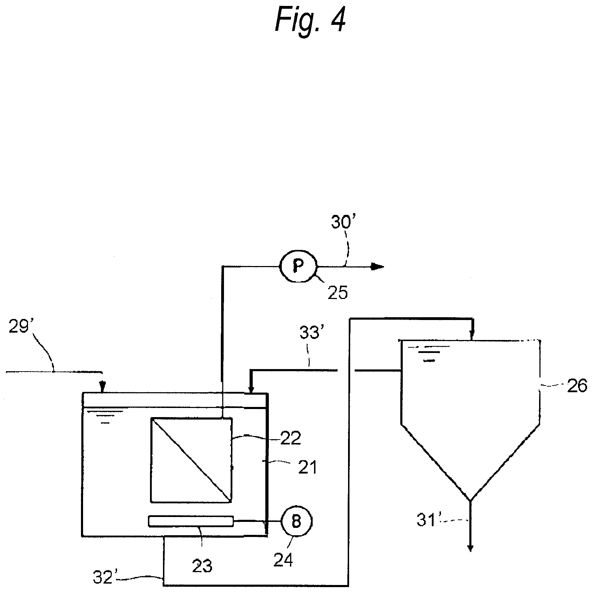

Fig. 4 is a diagram showing a second representative membrane separation step flow of a wastewater and a material flow in the production method of terephthalic acid. The second representative membrane separation step has the following constitutions.

(i) A wastewater 29' discharged from each of the steps in the production method of terephthalic acid is sent to the treatment tank 21.
(ii) The sent wastewater is separated into a separated water and a membrane-permeated water 30' by the membrane module 22.
(iii) The membrane-permeated water 30' is sent to the reverse osmosis membrane treatment step by a pump 25.
(iv) Aeration is performed from a lower part of the membrane module 22 through an air diffusion pipe 23 by a blower 24.
(v) A primary slurry 32' precipitated within the treatment tank 21 is sent to a thickener 26.
(vi) The sent primary slurry is again precipitated in the thickener 26.
(vii) A supernatant in an upper part of the thickener is returned as a returned water 33' to the treatment tank 21.
(viii) A slurry 31' precipitated in a lower part of the thickener is recovered.
(ix) The recovered slurry is sent to the oxidation reaction step.

Although a temperature in the inside of the thickener is not particularly limited, a temperature of the wastewater to be subjected to a membrane separation treatment is 40°C or higher and 60°C or lower.

When the case where as shown in Fig. 3, the membrane separation and the precipitation of the slurry are performed in the same tank, and the precipitation of slurry is performed in one stage is compared with the case where as shown in Fig. 4, the precipitation of the slurry is performed in two stages, the constitution of Fig. 4 provided with both the treatment tank and the thickener is more preferable from the standpoints that the slurry concentration is apt to become high, it is possible to make an average SS concentration of the entirety in the inside of the treatment tank low, and it is possible to make a size of one tank compact relative to the treatment amount of the wastewater.

(Reverse osmosis treatment step (8))

The reverse osmosis treatment step (8) is a step of separating the membrane-permeated water 27' into a treated water and a concentrated water by a reverse osmosis membrane. In the membrane-permeated wastewater 27', while the solid is removed, it still contains an organic compound such as terephthalic acid and p-toluic acid and an inorganic material that is a compound of a metal such as cobalt and manganese.

In this reverse osmosis membrane treatment step (8), the membrane-permeated water 27' is allowed to pass through the reverse osmosis membrane and separated into a permeated treated water and a non-permeated concentrated water. As for the content of impurities in the obtained treated water, a content of terephthalic acid is generally 10 ppm by weight or less; a total content of p-toluic acid and benzoic acid is generally 40 ppm by weight or less; a content of cobalt is generally 0.01 ppm by weight or less; a content of manganese is generally 0.01 ppm by weight or less; and a content of iron is generally 0.01 ppm by weight or less.

Examples of a material of the reverse osmosis membrane include polyethylene-based materials, polyamide-based materials inclusive of aromatic polyamide-based materials and crosslinked aromatic polyamide-based materials, aliphatic amine condensate-based materials, heterocyclic polymer-based materials, polyvinyl alcohol-based materials, and cellulose acetate-based materials. These materials may be used solely or in admixture of two or more kinds thereof. Of these, aromatic polyamide-based materials and polyamide-based materials inclusive of crosslinked aromatic polyamide-based materials are preferable because of high separation performance.

As a form of the reverse osmosis membrane, an asymmetric membrane and a composite membrane are exemplified, and these forms are selected solely or in admixture of two or more kinds thereof. Furthermore, a low-fouling membrane with an enhanced attachment-preventing performance to the surface of the reverse osmosis membrane is also preferably used. In addition, examples of the membrane module 22 of the reverse osmosis membrane may include a hollow fiber membrane type, a spiral membrane type, a tubular membrane type, a flat membrane type, a pleat type, and the like, and these types are selected solely or in admixture of two or more kinds thereof. Of these, a spiral membrane type module having a large membrane area and suitable for making the apparatus size compact is preferable. Specific examples thereof include "NTR-7250 (a registered trademark)" and "NTR-7410 (a registered trademark)" (all of which are manufactured by Nitto Denko Corporation), "FILMTEC (a registered trademark)" NF Series (manufactured by The Dow Chemical Company), and the like.

<B. Production method of terephthalic acid>

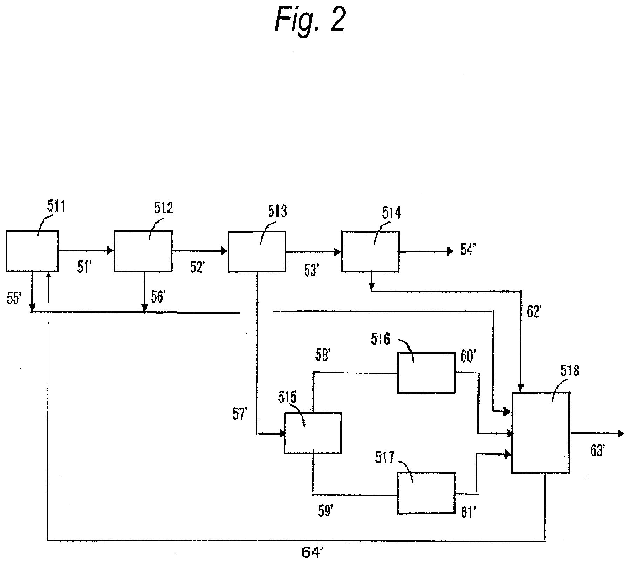

Fig. 2 is a diagram for explaining a step flow and a material flow in a second representative production method of terephthalic acid. The second representative production process of terephthalic acid includes the following 6 steps.

(9) Step of oxidizing p-xylene in aqueous acetic acid to obtain a crude terephthalic acid slurry (hereinafter sometimes referred to as "oxidation reaction step (9)");
(10) Step of further submitting the crude terephthalic acid slurry to an additional oxidation reaction under a high-temperature condition without feeing p-xylene, to obtain a terephthalic acid slurry (hereinafter sometimes referred to as "additional oxidation step (10)");

(11) Step of subjecting the terephthalic acid slurry to solid-liquid separation to obtain a terephthalic acid cake (hereinafter sometimes referred to as "solid-liquid separation step (11)");
(12) Step of drying the terephthalic acid slurry to obtain terephthalic acid (hereinafter sometimes referred to as "drying step (12)");
(13) Steps of recovering and regenerating valuables such as a catalyst and a solvent from a mother liquor after the solid-liquid separation (hereinafter sometimes referred to as "mother liquor recovery and regeneration step (13)");
(14) Step of subjecting the whole or a part of the wastewaters discharged from the steps (9) to (13) to solid-liquid separation with a separation membrane to obtain a membrane-permeated water (hereinafter sometimes referred to as "membrane separation step (14)"); and

(15) Step of separating the whole or a part of the membrane-permeated water obtained from the step (14) into a treated water and a mother liquor by a reverse osmosis membrane (hereinafter sometimes referred to as "reverse osmosis membrane treatment step (15)").

(Oxidation reaction step (9))

The oxidation reaction step (9) is a step of oxidizing p-xylene with a molecular oxygen-containing gas in aqueous acetic acid as a solvent in the presence of a catalyst in an oxidation reaction apparatus 511. Examples of the catalyst include a compound of a transition metal such as cobalt and manganese and/or a hydrogen halide such as hydrobromic acid, a bromine compound, and the like. Specifically, examples of the metal compound include cobalt acetate, manganese acetate, and the like. According to the oxidation reaction step (9), a crude terephthalic acid slurry 51' is obtained.

A reaction temperature is generally from 180°C to 230°C, and preferably from 190°C to 210°C. As for a reaction pressure, a pressure under which the mixture may keep a liquid phase at least at the reaction temperature, or more is needed, and specifically, it is preferably from 0.3 MPa to 10 MPa (absolute pressure), and more preferably from 1MPa to 3 MPa (absolute pressure).

From the oxidation reaction step (9), acetic acid as the solvent, water produced as a by-product in the reaction, other impurities, and a high-temperature high-pressure gas containing an unreacted raw material and the like are discharged while being entrained with the molecular oxygen-containing gas to be introduced into the oxidation reaction apparatus 511 for the oxidation reaction. The high-temperature high-pressure gas is allowed to pass through an absorption tower (not illustrated) or the like, and during that process, valuables, energy, and the like are recovered from the high-temperature high-pressure gas and submitted to reuse. Thereafter, the remaining gas is subjected to a combustion treatment. After an exhaust gas after the combustion treatment is subjected to contact washing with alkali water or the like, the gas is released to the air, and an oxidation exhaust gas washing water 55' is transferred into a treatment tank 518 having a separation membrane provided therein (hereinafter sometimes referred to as "treatment tank").

(Additional oxidation step (10))

The additional oxidation step (10) is a step in which the crude terephthalic acid slurry 51' obtained by the oxidation reaction is transferred into an additional oxidation reaction apparatus 512, and a molecular oxygen-containing gas is fed, whereby the crude terephthalic acid slurry 51' is further oxidized at a high temperature. According to the additional oxidation step (10), a terephthalic acid slurry 52' is obtained. A reaction temperature is generally from 235°C to 290°C, and preferably from 240°C to 280°C, and in general, a pressure is preferably from 3 MPa to 10 MPa (absolute pressure).

A part of the terephthalic acid particles in the crude terephthalic acid slurry 51' is dissolved, an oxidation intermediate, an unreacted raw material, and the like in the particles are oxidized, and an impurity concentration is reduced, thereby bringing a purification effect. The high-temperature high-pressure exhaust gas is treated in the same manner as that in the high-temperature high-pressure gas generated from the oxidation reaction step (9), and a remaining additional oxidation exhaust gas washing water 56' is transferred together with the oxidation exhaust gas washing water 55' into the treatment tank 518.

(Solid-liquid separation step (11) and mother liquor recovery and regeneration step (13))

The solid-liquid separation step (11) is a step of subjecting the terephthalic acid slurry 52' to solid-liquid separation in a solid-liquid separation apparatus 513, thereby obtaining a terephthalic acid cake 53'. Examples of a method of performing the solid-liquid separation may include a method of applying the terephthalic acid slurry 52' as it is to a solid-liquid separator, and the like. In addition, the terephthalic acid slurry 52' is in a pressurized state, and therefore, if the pressure is reduced, the dissolved terephthalic acid is deposited. For this reason, the terephthalic acid slurry 52' may be sent to a crystallization tank (not illustrated) to perform bursting and cooling and after depositing the dissolved terephthalic acid, applied to a solid-liquid separator. Incidentally, it is meant by the terms "bursting and cooling" that a target liquid is introduced into the crystallization tank set up under a pressure condition lower than the pressure of the target liquid, and by performing bursting in the crystallization tank, the resultant is cooled by means of expansion and vaporization of the solvent component.

From the solid-liquid separation step (11), the mother liquor and washing liquid having been subjected to solid-liquid separation (hereinafter sometimes referred to as "mother liquor and the like") are generated. 50% by weight or more of the mother liquor and the like are returned to the oxidation reaction step (9) and reused. Although a ratio of reusing the mother liquor and the like is preferably 70% by weight or more, it is needed to purge a part of the mother liquor for the purpose of preventing a lowering of the quality to be caused due to accumulation of impurities within the system. For that reason, the ratio of reusing the mother liquor is 95% by weight or less, and preferably 90% by weight or less. An oxidation reaction mother liquor (purge fraction) 57' which is not submitted to the reuse is sent to a solvent recovery apparatus 515 and submitted to the mother liquor recovery and regeneration step (13).

In the mother liquor recovery and regeneration step (13), the oxidation reaction mother liquor (purge fraction) 57' is evaporated and concentrated in the solvent recovery apparatus 515, whereby it is separated into an evaporated solvent 58' and a mother liquor-concentrated slurry 59' containing a valuable component. The mother liquor-concentrated slurry 59' is sent to a catalyst recovery/regeneration apparatus 517, whereby the catalyst is recovered/regenerated. After subjecting the recovered/regenerated catalyst to solid-liquid separation, a regenerated catalyst recovered and separated mother liquor 61' is sent together with a washing wastewater generated from a deodorization tower (not illustrated) to the treatment tank 518. Meanwhile, the evaporated solvent 58' is sent to a recovery apparatus 516 of acetic acid, etc. and allowed to pass through a dehydration tower (not illustrated), the acetic acid is concentrated and recovered, and subsequently, methyl acetate is recovered in a distillation tower (methyl acetate recovery tower) (not illustrated). A part of a remaining bottom liquid 60' in the methyl acetate recovery tower is sent to the treatment tank 518.

Incidentally, vent gases generated in other steps than the oxidation reaction step (9) and additional oxidation step (10) are collectively washed in a washing tower (not illustrated) and sent together with the oxidization exhaust gas washing water 55' and the additional oxidation exhaust gas washing water 56', and the like to the treatment tank 518.

(Drying step (12))

The drying step (12) is a step of drying a terephthalic acid cake 53' in a drying apparatus 514 to obtain terephthalic acid 54'. An exhaust gas generated from the drying apparatus 514 is washed in a washing tower (not illustrated), and a vent gas washing water 62' generated by washing is sent to the treatment tank 518.

(Membrane separation step (14))

An operation in the membrane separation step (14) is the same as the operation in the membrane separation step (7).

(Reverse osmosis membrane treatment step (15))

An operation in the reverse osmosis membrane treatment step (15) is the same as the operation in the reverse osmosis membrane treatment step (8).

Incidentally, in the present invention, even by either the above-described <A. Terephthalic acid production method> or the above-described <B. Terephthalic acid production method>, the same effects are brought so long as the terephthalic acid production method falls within the scope of the present invention.

Examples

The present invention is more specifically described below by reference to the following Example and Comparative Example, but it should not be construed that the present invention is limited to the following Examples.

[Measurement method of SS concentration (JIS K0102)]

As for a suspended substance (substance suspended in water), a sample is filtered, and a material remaining on a filter material is dried at 105°C to 110°C and measured for its mass according to the following operations.

(a) In the case of using a glass fiber filter paper, the glass fiber filter paper is installed in a filter in advance, and after thoroughly suction washing with water, this filter material is placed on a watch glass, heated at 105°C to 110°C for about 1 hour, and allowed to stand for cooling in a desiccator, followed by measuring its mass.
(b) The filter material is installed in the filter, and an appropriate amount of a sample is poured into the filter, followed by suction filtration. The substance attached to walls of a sample container and a filter pipe is washed off with water on the filter material, and the residual substances on the filter material are combined and washed several times with water.
(c) The residue is carefully taken out together with the filter material from the filter by using a pair of tweezers or the like, transferred onto the watch glass used in (a), heated at 105°C to 110°C for 2 hours, and allowed to stand for cooling in the preceding desiccator, followed by measuring its mass.
(d) An SS concentration (mg/L) is calculated according to the following equation <1>.

$$S = (a - b) \times 1000/V \qquad <1>$$

Here, S represents an SS concentration (mg/L); a represents a mass (mg) of the filter material and the watch glass containing the suspended substance; b represents a mass (mg) of the filter material and the watch glass; and V represents the sample (mL).

[Measurement method of cobalt concentration]

The cobalt concentration was measured using an ICP emission spectrophotometer in conformity with JIS K1020 60.3.

[Measurement method of manganese concentration]

The manganese concentration was measured using an ICP emission spectrophotometer in conformity with JIS K1020 56.4.

(Example 1)

In production equipment of terephthalic acid (production rate: 94 tons/hr), an oxidation reaction apparatus was continuously fed with p-xylene, acetic acid in an amount of 2.5 weight times the p-xylene, a mother liquor in an amount of 6.3 weight times the p-xylene, and cobalt acetate, manganese acetate, and hydrogen fluoride as catalysts, and an oxidation reaction was performed while adjusting a temperature at 195°C, a pressure at 1.5 MPa, and a reaction time (average residence time) at 90 minutes, respectively. As for use amounts of the catalysts, the amount of cobalt acetate was 300 ppm by weight in terms of a metal relative to the solvent, the amount of manganese acetate was 300 ppm by weight in terms of a metal relative to the solvent, and the amount of hydrogen bromide was 700 ppm by weight in terms of a metal relative to the solvent. Air was used as a molecular oxygen-containing gas for performing the oxidation reaction. At this time, an oxygen content of air is 21%.

Subsequently, the resultant was further subjected to an oxidation reaction with air that is the molecular oxygen-containing gas under conditions of a reaction temperature at 185°C, a pressure at 1.1 MPa, and a reaction time (average residence time) for 40 minutes, thereby obtaining a crude terephthalic acid slurry. The crude terephthalic acid slurry after completion of the reaction was subjected to solid-liquid separation by a screen bowl decanter while keeping the above-described reaction temperature and pressure, and the resulting crude terephthalic acid cake was washed with an acetic acid aqueous solution.

In addition, 80% by weight of the mother liquor obtained by solid-liquid separation was sent to the oxidation reaction apparatus and reused. Since the remaining 20% by weight fraction contains cobalt and manganese as active components, the solvent was subjected to evaporation to dryness, and a sodium carbonate aqueous solution was then added to convert cobalt and manganese into cobalt carbonate and manganese carbonate, respectively. Thereafter, acetic acid was added to these carbonates, thereby converting them into cobalt acetate and manganese acetate, respectively, which were then reused as the oxidation reaction catalysts. The residual wastewater from which the active components had been recovered was sent to the wastewater treatment step and after the treatment, was released to the outside. As for the crude terephthalic acid cake which had been washed with an acetic acid aqueous solution, the attached liquid was dried by a pressurized dryer, thereby obtaining crude terephthalic acid, which was then transferred into the subsequent hydrogenation treatment step.

In the hydrogenation treatment step, the crude terephthalic acid was mixed with water in a mixing tank, subsequently heated and pressurized at 290°C and 8.8 MPa with a vapor and a heat transfer oil, and then introduced together with hydrogen into a hydrogenation reaction tower having palladium-carbon as a fixed bed, thereby performing a hydrogenation reaction. The terephthalic acid aqueous solution after the reaction was continuously transferred into a crystallization tank and successively subjected to bursting and cooling to obtain a terephthalic acid slurry. Thereafter, the terephthalic acid slurry was subjected to solid-liquid separation at 160°C, and the resulting terephthalic acid cake was washed with water and dried. As for the dryer, a pressurized dryer and a fluidized bed dryer capable of performing bursting and cooling were used, and terephthalic acid was obtained in a yield of 94 tons/hr. In a separated mother liquor obtained by solid-liquid separation of the terephthalic acid slurry, organic materials such as terephthalic acid, p-toluic acid, and benzoic acid, and inorganic materials such as the metals derived from the catalysts are dissolved.

The wastewater from which the organic materials such as terephthalic acid had been separated from the slurry obtained by cooling the separated mother liquor at 52°C was sent to a treatment tank having a membrane module provided therein. Incidentally, an air diffuser for aeration is installed in a lower part of the membrane module.

An aggregate of polyvinylidene fluoride-made hollow fiber membranes having a pore diameter of 0.05 $\mu$m (membrane area: 1,536 m$^2$) was used as the membrane module. An aperture of an air discharge port of the air diffuser was $\phi$5 mm.

The wastewater sent to the treatment tank had an SS concentration of 10,000 mg/L and was subjected to a membrane separation treatment in the above-described treatment tank. Incidentally, a filtration amount of the wastewater per day by the membrane module was 3,840 m$^3$, and hence, a treatment amount of the wastewater per unit membrane area was 2.5 m/day. In addition, the aeration was fed at a rate of from 30 Nm$^3$/hr to 120 Nm$^3$/hr from the air diffuser in a lower part of the module. A linear velocity of the aeration was 80 m/hr. The membrane module underwent the membrane separation treatment while sucking the sent wastewater under a reduced pressure of from -10 KPa to -30 KPa.

The membrane-permeated water obtained by the membrane separation treatment was held in an interim tank and then sent to the reverse osmosis treatment step at a temperature of 40°C to 45°C while pressurizing at 0.8 to 1.2 MPa. In addition, the slurry precipitated in a lower part of the treatment tank was extracted, recovered from the lower part of the treatment tank, and returned to the oxidation reaction step, followed by reuse. Incidentally, a solid concentration of the slurry was 40% by weight.

In the reverse osmosis treatment step, "FILMTEC (a registered trademark)" (manufactured by The Dow Chemical Company) was used as the membrane module 22 that is the reverse osmosis membrane, and the membrane-permeated water was separated into a mother liquor and a treated water. The operation was performed at a flux of 0.75 m/day and in a weight ratio of the treated water to the concentrated water of 70/30. 38% by weight of the obtained treated water was used as the water for dissolving the crude terephthalic acid in the hydrogenation treatment step, and the remaining 62% by weight fraction was used as the terephthalic acid washing water during the solid-liquid separation.

Even after continuous operation for a half year or more, the product quality of produced terephthalic acid was stable. In addition, according to an analysis result of the treated water, the amount of terephthalic acid was 8 ppm by weight, the amount of p-toluic acid was 19 ppm by weight, the amount of benzoic acid was 8 ppm by weight, the amount of cobalt was 0.11 ppm by weight, and the amount of manganese was 0.03 ppm by weight, respectively in average; and iron was not detected, and the amount of bromine was 3 ppm by weight or less.

The concentrated water separated by the reverse osmosis membrane was sent to a metal recovery step, and the metals derived from the catalysts were recovered. The mother liquor after recovering the metals was sent to a product treatment step, and 60% of the amount of the wastewater was subjected to water recovery. The recovered water was used as a cooling water for various instruments. Incidentally, the amount of the wastewater released was 53 m$^3$/hr.

(Comparative Example)

In production equipment of terephthalic acid (production rate: 94 tons/hr), an oxidation reaction apparatus was continuously fed with p-xylene, acetic acid in an amount of 2.5 weight times the p-xylene, a mother liquor in an amount of 6.3 weight times the p-xylene, and cobalt acetate, manganese acetate, and hydrogen fluoride as catalysts, and an oxidation reaction was performed at a temperature of 195°C and a pressure of 1.5 MPa for a reaction time (average residence time) of 90 minutes.

As for use amounts of the catalysts, the amount of cobalt acetate was 300 ppm by weight in terms of a metal relative to the solvent, the amount of manganese acetate was 300 ppm by weight in terms of a metal relative to the solvent, and the amount of hydrogen bromide was 700 ppm by weight in terms of a metal relative to the solvent. Air was used as a molecular oxygen-containing gas for performing the oxidation reaction. At this time, an oxygen content of air is 21%. A crude terephthalic acid slurry was obtained by the above-described oxidation reaction.

Subsequently, the crude terephthalic acid slurry was further subjected to an oxidation reaction with air that is the molecular oxygen-containing gas under conditions of a reaction temperature at 185°C, a pressure at 1.1 MPa, and a reaction time (average residence time) for 40 minutes, thereby obtaining a crude terephthalic acid slurry. The crude terephthalic acid slurry after completion of the reaction was subjected to solid-liquid separation by a screen bowl decanter while keeping the above-described reaction temperature and pressure, and the resulting crude terephthalic acid cake was washed with an acetic acid aqueous solution.

In addition, 80% by weight of the mother liquor obtained by solid-liquid separation was sent to the oxidation reaction apparatus and reused. Since the remaining 20% by weight fraction contains cobalt and manganese as active components, the solvent was subjected to evaporation to dryness, and a sodium carbonate aqueous solution was then added to convert cobalt and manganese into cobalt carbonate and manganese carbonate, respectively. Thereafter, acetic acid was added to these carbonates, thereby converting them into cobalt acetate and manganese acetate, respectively, which were then reused as the oxidation reaction catalysts. The residual wastewater from which the active components had been recovered was sent to the wastewater treatment step and after the treatment, was released to the outside. As for the crude terephthalic acid cake which had been washed with an acetic acid aqueous solution, the attached liquid was dried by a pressurized dryer, thereby obtaining crude terephthalic acid, which was then transferred into the subsequent hydrogenation treatment step.

In the hydrogenation treatment step, the crude terephthalic acid was mixed with water in a mixing tank, subsequently heated and pressurized at 290°C and 8.8 MPa with a vapor and a heat transfer oil, and then introduced together with hydrogen into a hydrogenation reaction tower having palladium-carbon as a fixed bed, thereby performing a hydrogenation reaction. The terephthalic acid aqueous solution after the reaction was continuously transferred into a crystallization tank and successively subjected to bursting and cooling to obtain a terephthalic acid slurry. Thereafter, the terephthalic acid slurry was subjected to solid-liquid separation at 160°C, and the resulting terephthalic acid cake was washed with water and dried. As for the dryer, a pressurized dryer and a fluidized bed dryer capable of performing bursting and cooling were used, and terephthalic acid was obtained in a yield of 75 tons/hr. In a separated mother liquor obtained by solid-liquid separation of the terephthalic acid slurry, organic materials such as terephthalic acid, p-toluic acid, and benzoic acid, and inorganic materials such as the metals derived from the catalysts are dissolved.

The wastewater from which the organic materials such as terephthalic acid had been separated from the slurry obtained by cooling the separated mother liquor at 52°C was sent to a treatment tank having a membrane module provided therein.

An aggregate of polyvinylidene fluoride-made hollow fiber membranes having a pore diameter of 0.05 $\mu$m (membrane area: 1,536 m$^2$) was used as the membrane module.

The wastewater sent to the treatment tank had an SS concentration of 5,000 ppm by weight and was subjected to a membrane separation treatment in the above-described treatment tank. The membrane module underwent the membrane separation treatment while sucking the sent wastewater under a reduced pressure of from -10 KPa to -30 KPa. Precipitation of the slurry relative to an increase of the solid within the treatment tank to be caused due to the membrane separation treatment was so slow that it became impossible to continue the membrane separation treatment within a short period of time (10 minutes).

Industrial Applicability

According to the production method of terephthalic acid of the present invention, a high-concentration slurry can be efficiently recovered from a wastewater containing turbidity, which is discharged from a terephthalic acid production process, and it becomes possible to reduce the turbidity of the wastewater in the neighborhood of a separation membrane for performing filtration of the wastewater, thereby making it possible to undergo a wastewater treatment continuously and stably over a long period of time. Furthermore, by reusing the high-concentration slurry for the terephthalic acid production process, terephthalic acid with enhanced production efficiency can be stably produced and can be suitably used during industrial production of terephthalic acid.

While the invention has been described in detail and with reference to specific embodiments thereof, it will be apparent to one skilled in the art that various changes and modifications can be made therein without departing from the spirit and scope thereof. This application is based on a Japanese patent application filed on February 27, 2014 (Application No. 2014-037215), the content thereof being incorporated herein by reference.

Explanations of Reference Signs

| 11: | Oxidation reaction apparatus |
| 12: | First solid-liquid separation apparatus |
| 13: | Hydrogenation treatment apparatus |
| 14: | Crystallization apparatus |
| 15: | Second solid-liquid separation apparatus |
| 16: | Drying apparatus |
| 17: | Solvent recovery apparatus |
| 18: | Recovery apparatus of acetic acid, etc. |
| 19: | Catalyst recovery/regeneration apparatus |
| 20: | Recovery apparatus of p-toluic acid, etc. |
| 21: | Treatment tank |
| 22: | Membrane module |
| 23: | Air diffusion pipe |
| 24: | Blower |
| 25: | Pump |
| 26: | Thickener |
| 11': | Crude terephthalic acid slurry |
| 12': | Crude terephthalic acid |
| 13': | Hydrogenation treatment liquid |
| 14': | Terephthalic acid slurry |
| 15': | Terephthalic acid cake |
| 16': | Terephthalic acid |
| 17': | Oxidization exhaust gas washing water |
| 18': | Oxidation reaction mother liquor (purged fraction) |
| 19': | Evaporated solvent |
| 20': | Mother liquor concentrated slurry |
| 21': | Bottom liquid in methyl acetate recovery tower |
| 22': | Regenerated catalyst recovered and separated mother liquor |
| 23': | Condensed water generated at the time of crystallization |
| 24': | Terephthalic acid-separated mother liquor |
| 25': | Separated mother liquor of p-toluic acid, etc. |
| 26': | Vent gas washing water |
| 27': | Membrane-permeated water |
| 28': | Slurry |
| 29': | Wastewater |
| 30': | Membrane-permeated water |
| 31': | Slurry |
| 32': | Primary slurry |
| 33': | Returned water |
| 511: | Oxidation reaction apparatus |
| 512: | Additional oxidation reaction apparatus |
| 513: | Solid-liquid separation apparatus |
| 514: | Drying apparatus |
| 515: | Solvent recovery apparatus |
| 516: | Recovery apparatus of acetic acid, etc. |
| 517: | Catalyst recovery/regeneration apparatus |
| 518: | Treatment apparatus |
| 51': | Crude terephthalic acid slurry |
| 52': | Terephthalic acid slurry |
| 53': | Terephthalic acid cake |
| 54': | Terephthalic acid |
| 55': | Oxidation exhaust gas washing water |
| 56': | Additional oxidation exhaust gas washing water |
| 57': | Oxidation reaction mother liquor (purged fraction) |

| 58': | Evaporated solvent |
| 59': | Mother liquor-concentrated slurry |
| 60': | Bottom liquid in methyl acetate recovery tower |
| 61': | Regenerated catalyst recovered and separated mother liquor |
| 62': | Vent gas washing water |
| 63': | Membrane-permeated water |
| 64': | Slurry |

**Claims**

1. A method for producing terephthalic acid comprising a membrane separation step of subjecting a wastewater discharged from a terephthalic acid production process including an oxidation reaction step of oxidizing p-xylene in the presence of a catalyst to form crude terephthalic acid, to a membrane separation treatment in a treatment tank having a separation membrane provided therein, thereby separating it into a membrane-permeated water and a separated water which is not permeated by the separation membrane, wherein aeration is performed from a lower part of the separation membrane.

2. The method for producing terephthalic acid according to claim 1, wherein the membrane separation step includes a membrane separation treatment step and a step of precipitating a slurry.

3. The method for producing terephthalic acid according to claim 1 or 2, wherein a temperature of the wastewater to be subjected to a membrane separation treatment is 30°C or higher and 60°C or lower.

4. The method for producing terephthalic acid according to any one of claims 1 to 3, wherein a linear velocity of a flowing of the wastewater on the surface of the separation membrane is 50 m/hr or more and 200 m/hr or less.

5. The method for producing terephthalic acid according to any one of claims 1 to 4, wherein a suspended solid (SS) concentration of the wastewater to be subjected to a membrane separation treatment is 1,000 mg/L or more and 15,000 mg/L or less.

6. The method for producing terephthalic acid according to any one of claims 1 to 5, wherein the slurry is recovered from the separated water.

7. The method for producing terephthalic acid according to claim 6, wherein the recovered slurry is used as a process water of the terephthalic acid production process.

8. The method for producing terephthalic acid according to any one of claims 1 to 7, wherein the aeration is performed by an air diffuser.

9. The method for producing terephthalic acid according to claim 8, wherein an air discharge port is provided in the air diffuser, and an aperture of the air discharge port is $\phi$1 mm or more and $\phi$10 mm or less.

10. The method for producing terephthalic acid according to any one of claims 1 to 9, wherein the separation membrane is a membrane filter.

11. The method for producing terephthalic acid according to any one of claims 1 to 10, wherein a pore diameter of the separation membrane is from 0.01 $\mu$m to 10 $\mu$m.

12. The method for producing terephthalic acid according to any one of claims 1 to 11, wherein the separation membrane is a hollow fiber membrane.

13. The method for producing terephthalic acid according to any one of claims 1 to 12, wherein a membrane separation treatment amount (m/day) per unit membrane area (m$^2$) in the separation membrane is 0.5 or more and 5 or less.

# Fig. 1

# Fig. 2

# Fig. 3

# Fig. 4

| **INTERNATIONAL SEARCH REPORT** | International application No. |
| --- | --- |
| | PCT/JP2015/055717 |

| A. CLASSIFICATION OF SUBJECT MATTER |
| --- |
| *C07C51/42*(2006.01)i, *C07C63/26*(2006.01)i |

According to International Patent Classification (IPC) or to both national classification and IPC

| B. FIELDS SEARCHED |
| --- |
| Minimum documentation searched (classification system followed by classification symbols)<br>C07C51/42, C07C63/26 |

Documentation searched other than minimum documentation to the extent that such documents are included in the fields searched

| | |
| --- | --- |
| Jitsuyo Shinan Koho | 1922–1996 Jitsuyo Shinan Toroku Koho 1996–2015 |
| Kokai Jitsuyo Shinan Koho | 1971–2015 Toroku Jitsuyo Shinan Koho 1994–2015 |

Electronic data base consulted during the international search (name of data base and, where practicable, search terms used)

| C. DOCUMENTS CONSIDERED TO BE RELEVANT |
| --- |

| Category* | Citation of document, with indication, where appropriate, of the relevant passages | Relevant to claim No. |
| --- | --- | --- |
| X<br>A | JP 2014-5226 A (Mitsubishi Chemical Corp.),<br>16 January 2014 (16.01.2014),<br>paragraphs [0062] to [0065]; examples; fig. 1,<br>6<br>(Family: none) | 1-6,8-13<br>7 |
| Y | JP 2004-175797 A (Mitsubishi Chemical Corp.),<br>24 June 2004 (24.06.2004),<br>paragraphs [0063] to [0077]; fig. 1<br>& US 2006/0014979 A1 | 1-13 |
| Y | JP 2001-328957 A (Mitsui Chemicals, Inc.),<br>27 November 2001 (27.11.2001),<br>fig. 4<br>(Family: none) | 1-13 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>30 April 2015 (30.04.15) | Date of mailing of the international search report<br>19 May 2015 (19.05.15) |
| --- | --- |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (July 2009)

## INTERNATIONAL SEARCH REPORT

International application No.

PCT/JP2015/055717

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2013-240794 A (Kobelco Eco-Solutions Co., Ltd.), 05 December 2013 (05.12.2013), paragraphs [0069], [0082]; fig. 1 (Family: none) | 1-13 |
| Y | JP 2010-207657 A (Hitachi Plant Technologies, Ltd.), 24 September 2010 (24.09.2010), paragraphs [0056], [0057]; fig. 1 (Family: none) | 1-13 |
| A | WO 2006/100969 A1 (Mitsubishi Chemical Corp.), 28 September 2006 (28.09.2006), paragraphs [0033], [0042] to [0044], [0055], [0064]; fig. 1 & US 2009/0018361 A1 & EP 1862445 A1 | 1-13 |
| E,X E,A | JP 2015-71155 A (Mitsubishi Rayon Co., Ltd.), 16 April 2015 (16.04.2015), paragraphs [0139], [0144]; fig. 1, 10, 11, 13 (Family: none) | 1-6,8-13 7 |

Form PCT/ISA/210 (continuation of second sheet) (July 2009)

**EP 3 112 342 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5056329 B
- JP 3372921 B
- CN 100400432 C
- CN 100544429 B
- CN 101723531 B
- JP 2014037215 A